Europäisches Patentamt

European Patent Office

Office européen des brevets

(11) Publication number : **0 499 480 A1**

# EUROPEAN PATENT APPLICATION

(21) Application number : 92301228.0

(22) Date of filing : 14.02.92

(51) Int. Cl.⁵ : **A61F 2/36, A61F 2/30**

(30) Priority : **15.02.91 GB 9103248**

(43) Date of publication of application :
**19.08.92 Bulletin 92/34**

(84) Designated Contracting States :
**AT BE CH DE DK ES FR GB GR IT LI LU NL PT
SE**

(71) Applicant : **AESCULAP LIMITED
43 Allen Street
Sheffield S3 7AX (GB)**

(72) Inventor : **Field, Richard Eddy
23 Redston Road
London N8 7HL (GB)**

(74) Representative : **Houghton, David et al
Hulse & Co. Eagle Star House, Carver Street
Sheffield S1 4FP (GB)**

(54) **Hip implants.**

(57)   The invention relates to femoral components for use in hip replacement surgery. It comprises a tail (distal) section (2) having transversely convex lateral (3) and medial (4) surfaces of the same diameter, extending to a head end (proximal) part (5) having first and second sections (6,7), said first and second sections (6,7) having a transversely convex lateral surface (8) the apexes of which are co-planar with the apex of the lateral surface (4) of the tail section, the diameter of lateral surface (8) of the first section (6) progressively increasing to the second section (7), the lateral surface of which is of constant diameter from its junction with the first section to the end (9) of the component, and the medial surfaces (10,11) of the first and second sections (6,7) each being continuously longitudinally concave and laterally convex, side surfaces (12,13) of the first and second sections (6,7) lying tangentially to the convex lateral surfaces (8) and with the angle of the tangent over the first section (6) progressively decreasing in the direction towards the second section (7).

Fig. 1

EP 0 499 480 A1

This invention relates to artificial implants, and in particular to a femoral component for use in hip replacement surgery.

As is well-known in the art, hip replacement surgery involves the severing of a worn or damaged ball-head from the end of a femur, the fashioning of a cavity extending down the femur shaft, and the securing in the cavity of a femoral component. As is also well-known in the art, femoral components are essentially of the two types, those that are secured in the cavity by an appropriate cement, and those that are a press fit in the cavity. Whilst with femoral components of both types considerable attention has to be paid to ensuring a close co-operation as between the component and the cavity such considerations are less exacting with cemented components than with press fit components. With the latter, not only must there be contact between the component and cavity formed in the femur, but also there must be avoided any localised stressing of the femur and of the component that could lead to the subsequent failure of the component or fracturing of the femur.

Another important consideration is to keep to a minimum the removal of healthy bone from within the proximal end of the femur and yet have a component located within the cavity of sufficient bulk to withstand all loads imposed during normal activities undertaken by the patient, so as to prevent movement of the implant by rotation or subsidence that would lead to pain and eventual failure by loosening at the implant/bone interface.

The object of the invention is to provide a femoral component that meets those requirements mentioned above.

According to the present invention, a femoral component for use in hip replacement comprises a tail (distal) section having transversely convex lateral and medial surfaces of the same diameter, extending to a head end (proximal) part having first and second sections, said first and second sections having a transversely convex lateral surface the apexes of which are co-planar with the apex of the lateral surface of the tail section, the diameter of lateral surface of the first section progressively increasing to the second section, the lateral surface of which is of constant diameter from its junction with the first section to the end of the component, and the medial surfaces of the first and second sections each being continuously longitudinally concave and laterally convex, side surfaces of the first and second sections lying tangentially to the convex lateral surfaces and with the angle of the tangent over the first section progressively decreasing in the direction towards the second section. Preferably, the medial surfaces of first and second sections have the same diameter as that of the medial surface of the tail (distal) section at its junction with the first section. The tail (distal) section may be circular and may be tapered from the junction with the first section down to its bottom end. Preferably, however, the tail (distal) section is of constant cross-section along its length and formed by convex lateral and medial surfaces spaced by parallel anterior/posterior faces that merge into the anterior/posterior faces of the first section of the hip implant.

In its basic construction the femoral component may be secured in an appropriately formed cavity by cement. To enable the component to be secured as a press fit, longitudinal grooves may be provided parallel to each other and extending down the side surfaces of the first and second sections of the head (proximal) end of the component, the depths of which progressively decrease to the point where they merge with the concave medial surface of the component.

The invention, particularly in its press fit form of construction, takes cognisance of the fact that an efficient means of creating a cavity in the femur with minimal bone removal, is to first drill a circular hole axially down the femur, and then to generate a slot extending from the hole with a concave medial surface, and such as is described in co-pending Application 9101598.2. The femoral component of the present invention can then be inserted into the cavity so formed, and with the tail (distal) section in its preferred form fitting so as to guide on its medial and lateral aspects whilst not touching on its anterior and posterior surfaces, and with the first and second sections of the anterior and posterior surfaces fitting as an interference fit in the slot extending from the distal hole. By constructing the femoral component with a longitudinally concave medial surface along the proximal end to the same radius as the medial surface formed in the slot extending from the hole, abutting contact over the full length of the said medial surface is provided once the femoral component is driven into the cavity in the femur.

The presence of longitudinal grooves down the outwardly diverging anterior/posterior faces of the first and second sections of the proximal end is of considerable benefit in the securing of the component, easing insertion of the implant and preventing splitting of the femur, consequent on the grooves allowing the ingress of bone as the lands bite into the surfaces of the slot extending from the hole.

The femoral component of the invention lends itself to efficient and cost-effective production.

Femurs can be generalised as falling into two categories, one displaying a relatively large medial radius with the axis of the neck of the femur at a relatively steep angle, and the other displaying a relatively small medial radius with the axis of the neck of the femur at a relatively shallow angle. The production of blanks can all be to suit femurs of the first category, and when a component is needed to suit femurs of the second category, a required deeper medial radius ground on to it with its consequential effect of reducing the angle of the axis of the trunnion on which the head of the component sits.

Two embodiments of the invention will now be described with reference to the accompanying drawings, in which:

Figure 1 is a perspective view of one embodiment of femoral component in accordance with the invention;

Figure 2 is a section on the line II-II of Figure 1;

Figure 3 is a section on the line III-III of Figure 1;

Figure 4 is a section on the line IV-IV of Figure 1;

Figure 5 is a portrayal of the femoral component of Figure 1 located within a femur; and

Figure 6 corresponds to Figure 5 but shows an alternative embodiment of femoral component intended to be located in a femur by an appropriate cement.

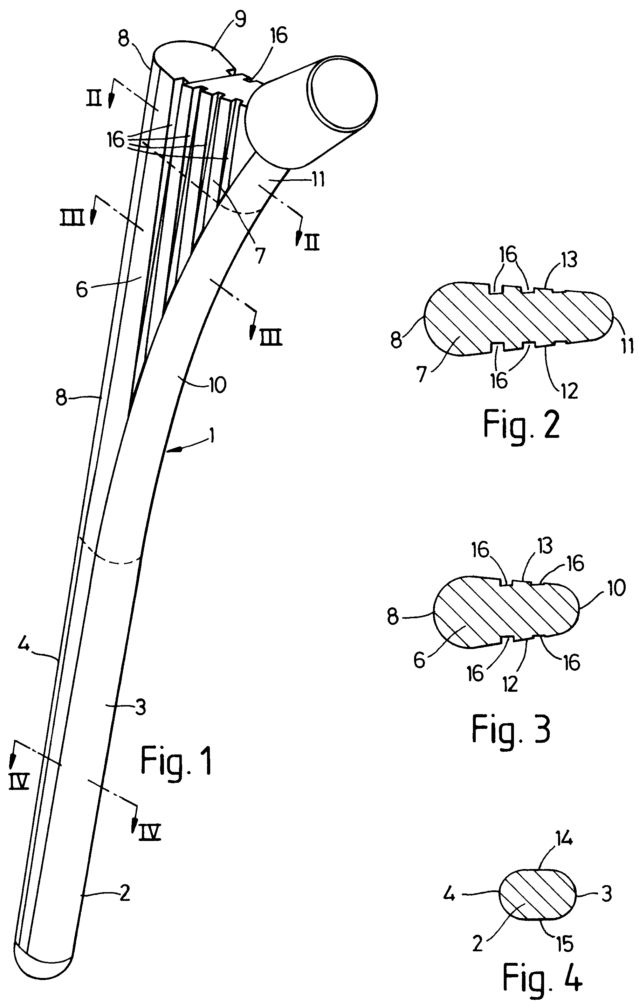

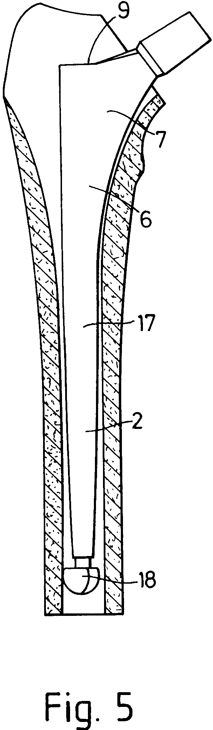

In Figures 1 to 5, a femoral component 1 for use in hip replacement has a tail (distal) section 2 having transversely convex, lateral and medial surfaces 3, 4 of the same diameter, extending to a head (proximal) end part 5 having first and second sections 6, 7 each having a laterally convex surface 8, the apex of which is co-planar with the apex of the lateral surface 4 of the tail section, the diameter of the lateral surface 8 of the first section 6 progressively increasing in the direction towards the second section 7 and the lateral surface of the second section 7 being of constant diameter from its junction with the first section 6 to the end 9 of the component. The medial surfaces 10, 11 of the first and second sections 6, 7 are each continuously longitudinally concave, and laterally convex and have the same diameter as that of the medial surface 3 of the tail (distal) section 2 at its junction with the first section 6. The side surfaces 12, 13 of the first and second sections are so arranged as to be tangential to the convex lateral surfaces of the first and second sections, and consequently the angle of the tangent over the length of the first section progressively decreases in the direction towards the second section 7.

To facilitate insertion of the femoral component into a hole drilled centrally down a femur, it is preferred that the tail (distal) section 2 is of constant cross-section along its length and with the convex lateral and medial surfaces 4, 3 spaced by parallel anterior and posterior faces 14, 15 that merge into the anterior and posterior faces 12, 13 of the first section.

To ensure that the component can be effectively secured as a press fit in the hole formed in the femur, longitudinal, parallel grooves 16 are formed in the anterior and posterior side surfaces of the first and second sections 6, 7, the depths of the grooves progressively decreasing in a direction away from the end 9 of the component to the point where they merge with the concave medial surfaces 10, 11 of the first and second sections 6, 7.

As is indicated by Figure 6, a femoral component 17 can be provided, the structure and cross-section of which across its tail 2, first and second sections 6, 7, are precisely as are defined in connection with the femoral component 1. In this case, however, the femoral component 17 is to be located and secured in the hole formed in the femur by an appropriate cement. Thus, with this construction, the presence of parallel grooves 16 has been dispensed with, and the tail section 2 is tapered along its length and terminates with a cement spacer 18. Thus cement can be introduced to fill the gap existing between the component and the hole to secure the femoral component in place, centrally within the reamed medullary canal.

## Claims

1. A femoral component for use in hip replacement characterised by a tail (distal) section (2) having transversely convex lateral (3) and medial (4) surfaces of the same diameter, extending to a head end (proximal) part (5) having first and second sections (6, 7), said first and second sections (6, 7) having a transversely convex lateral surface (8) the apexes of which are co-planar with the apex of the lateral surface (4) of the tail section, the diameter of lateral surface (8) of the first section (6) progressively increasing to the second section (7), the lateral surface of which is of constant diameter from its junction with the first section to the end (9) of the component, and the medial surfaces (10, 11) of the first and second sections (6, 7) each being continuously longitudinally concave and laterally convex, side surfaces (12, 13) of the first and second sections (6, 7) lying tangentially to the convex lateral surfaces (8) and with the angle of the tangent over the first section (6) progressively decreasing in the direction towards the second section (7).

2. A femoral component as in Claim 1, characterised in that the medial surfaces (10, 11) of first and second sections (6, 7) have the same diameter as that of the medial surface (4) of the tail (distal) section (2) at its junction with the first section.

3. A femoral component as in Claim 1 or Claim 2, characterised in that the tail (distal) section (2) is circular and tapers towards its bottom end.

4. A femoral component as in Claim 1 or Claim 2, characterised in that the tail (distal) section (2) is of constant cross-section along its length and formed by convex lateral and medial surfaces (3, 4) spaced by parallel anterior/posterior faces that merge into the anterior/posterior faces (12, 13) of the first section of the hip implant.

5. A femoral component as in any of Claims 1 to 4, characterised in that to enable the component to be secured as a press fit, longitudinal grooves (16) are provided parallel to each other, and extending down the anterior and posterior side surfaces (12, 13), said grooves having depths that progressively decrease to the point where they merge with the concave medial surfaces (10, 11) of the first and second sections (6, 7).

6. A femoral component as in any of Claims 1 to 4, characterised in that to enable the component to be secured by cement, and to be located centrally of a hole formed axially along the femur, the tail (distal) end (2) is of circular section and tapered and terminates in a cement spacer (18).

Fig. 1

Fig. 2

Fig. 3

Fig. 4

Fig. 5

EP 0 499 480 A1

**European Patent Office**

# EUROPEAN SEARCH REPORT

Application Number

EP 92 30 1228

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 638 962 (LA BIOMECANIQUE INTEGREE) | 1,2 | A61F2/36 |
| Y | * page 3, line 11 - page 4, line 34; figures * | 3-6 | A61F2/30 |
| | --- | | |
| Y | EP-A-0 135 755 (PROTEK AG) | 4,5 | |
| | * page 2, line 26 - page 4, line 15; figures * | | |
| | --- | | |
| Y | EP-A-0 363 151 (PFIZER HOSPITAL) | 3,6 | |
| | * page 6, line 14 - page 7, line 57; figures 1-14 * | | |
| | --- | | |
| A | FR-A-2 639 821 (FABRIQUE D'IMPLANTS ET D'INSTRUMENTS CHIRURGICAUX) * abstract; figures * | 1 | |
| | --- | | |
| A | FR-A-2 602 672 (MARQUER ET AL) | | |
| | --- | | |
| A | FR-A-2 636 837 (G. CREMASCOLI) | | |
| | ----- | | **TECHNICAL FIELDS SEARCHED (Int. Cl.5)** |
| | | | A61F |

The present search report has been drawn up for all claims

| Place of search | Date of completion of the search | Examiner |
|---|---|---|
| THE HAGUE | 31 MARCH 1992 | SANCHEZ Y SANCHEZ J. |

EPO FORM 1503 03.82 (P0401)